# EUROPÄISCHE PATENTSCHRIFT

(11) **EP 3 658 200 B1**
(45) Veröffentlichungstag und Bekanntmachung des Hinweises auf die Patenterteilung: **07.09.2022**
(21) Anmeldenummer: 18740762.2
(22) Anmeldetag: 06.07.2018
(51) Int. Cl.: A61M 1/14, H04B 10/80

(54) **MEDIZINTECHNISCHES GERÄT MIT EINER FÜR DEN SCHUTZ VON PATIENTEN UND BEDIENER EINGERICHTETEN KOMMUNIKATIONSSCHNITTSTELLE**
MEDICAL DEVICE HAVING A COMMUNICATION INTERFACE DESIGNED FOR PROTECTING PATIENTS AND OPERATORS
APPAREIL MÉDICAL MUNI D'UNE INTERFACE DE COMMUNICATION CONÇUE POUR LA PROTECTION DES PATIENTS ET DE L'OPÉRATEUR

(30) Priorität: 27.07.2017 DE 102017007033
(43) Veröffentlichungstag der Anmeldung: 03.06.2020
(73) Patentinhaber: Fresenius Medical Care Deutschland GmbH, 61352 Bad Homburg (DE)
(72) Erfinder: SAAL, Stefan, 97453 Schonungen (DE); HEDMANN, Frank, 97332 Volkach (DE); BUDA, Martin, 97456 Dittelbrunn (DE); HOCHREIN, Torsten, 97478 Eschenau (Knetzgau) (DE)
(74) Vertreter: Hitzelberger, Christoph Michael
(86) Internationale Anmeldenummer: PCT/EP2018/068325
(87) Internationale Veröffentlichungsnummer: WO 2019/020343

(56) Entgegenhaltungen:
- US-A- 3 910 257
- US-A1- 2004 113 498
- US-A1- 2009 256 527
- US-A1- 2015 097 701

## Beschreibung

Die vorliegende Beschreibung bezieht sich auf medizintechnisches Gerät mit einer Kommunikationsschnittstelle, die für den Schutz von Patienten und Bediener vor gefährlichen elektrischen Spannungen und Strömen und für eine geringe Empfindlichkeit gegenüber Störungen eingerichtet ist.

### Hintergrund

Medizintechnische Geräte sind zunehmend mit Schnittstellen zur Kommunikation über eine Netzwerk-Kommunikations-Infrastruktur ausgestattet, zum Beispiel um während des Betriebs erfasste Geräte- und Patientendaten an Datenbanken oder Server zur Speicherung bzw. Verarbeitung zu übermitteln, oder um eine Fernüberwachung und -steuerung zu ermöglichen, aber auch um Fernwartungsfunktionen wie bspw. das Aktualisieren einer Steuersoftware oder von Betriebsparametern zu ermöglichen.

Medizintechnische Geräte umfassen bspw. Dialysegeräte, Geräte zur Überwachung von Kreislaufparametern von Patienten, Infusionsgeräte und dergleichen, siehe z.B. US 2009/256527.

Dialysegeräte sind Geräte zur extrakorporalen Blutbehandlung, bei denen Blut eines Patienten über eine erste Fluidleitung einer Blutbehandlungskomponente zugeführt, durch die Blutbehandlungskomponente behandelt und über eine zweite Fluidleitung wieder an den Patienten zurückgegeben wird. Beispiele für solche Blutbehandlungsgeräte sind insbesondere Hämodialysegeräte. Ein solches Blutbehandlungsgerät ist Gegenstand der DE 198 49 787 C1 der Anmelderin.

Die Dialyse ist ein Verfahren zur Blutreinigung für Patienten mit akuter oder chronischer Niereninsuffizienz. Grundsätzlich unterscheidet man hierbei zwischen Verfahren mit einem extrakorporalen Blutkreislauf, wie der Hämodialyse, der Hämofiltration oder der Hämodiafiltration und der Peritonealdialyse, die keinen extrakorporalen Blutkreislauf aufweist.

Das Blut wird bei der Hämodialyse in einem extrakorporalen Blutkreislauf durch die Blutkammer eines Dialysators geleitet, die über eine semipermeable Membran von einer Dialysierflüssigkeitskammer getrennt ist. Die Dialysierflüssigkeitskammer wird von einer Blutelektrolyte in einer bestimmten Konzentration enthaltenen Dialysierflüssigkeit durchströmt. Die Stoffkonzentration der Blutelektrolyte in der Dialysierflüssigkeit entspricht dabei der Konzentration der Blutelektrolyte im Blut eines Gesunden. Während der Behandlung werden das Blut des Patienten und die Dialysierflüssigkeit an beiden Seiten der semipermeablen Membran im Allgemeinen im Gegenstrom mit einer vorgegebenen Flussrate vorbeigeführt. Harnpflichtige Stoffe diffundieren durch die Membran von der Blutkammer in die Kammer für Dialysierflüssigkeit, während im Blut und in der Dialysierflüssigkeit vorhandene Elektrolyte jeweils von der Kammer höherer Konzentration zur Kammer niedrigerer Konzentration diffundieren. Wird an der Dialysemembran ein Druck¬gradient von der Blutseite zur Dialysatseite aufgebaut, beispielsweise durch eine Pumpe, die flussabwärts des Dialysefilters auf der Dialysatseite Dialysat aus dem Dialysatkreislauf entzieht, tritt Wasser aus dem Patientenblut über die Dialysemembran in den Dialysatkreislauf über. Dieser auch als Ultrafiltration bezeichnete Vorgang führt zu einer gewünschten Entwässerung des Patientenbluts.

Bei der Hämofiltration wird dem Patientenblut durch Anlegen eines Transmembrandrucks im Dialysator Ultrafiltrat entzogen, ohne dass Dialysierflüssigkeit auf der dem Patientenblut gegenüber liegenden Seite der Membran des Dialysators vorbeigeführt wird. Zusätzlich kann dem Patientenblut eine sterile und pyrogenfreie Substituatlösung zugesetzt werden. Je nachdem, ob diese Substituatlösung stromaufwärts des Dialysators zugesetzt wird oder stromabwärts, spricht man von Prä- oder Postdilution. Der Stoffaustausch erfolgt bei der Hämofiltration konvektiv.

Die Hämodiafiltration kombiniert die Verfahren der Hämodialyse und der Hämofiltration. Es findet sowohl ein diffusiver Stoffaustausch zwischen Patientenblut und Dialysierflüssigkeit über die semipermeable Membran eines Dialysators statt, als auch eine Abfiltrierung von im Blut enthaltenem Plasmawasser durch einen Druckgradienten an der Membran des Dialysators.

Die Verfahren der Hämodialyse, der Hämofiltration und der Hämodiafiltration werden in der Regel mit automatischen Hämodialysegeräten durchgeführt, wie sie beispielsweise von der Anmelderin vertrieben werden.

Die Entnahme des Blutes aus dem Patienten und Einbringung in den extrakorporalen Blutkreislauf erfolgt mittels Blutpumpen, bspw. Schlauchrollenpumpen oder Impellerpumpen, welche das Blut aus dem Blutkreislauf des Patienten und zum Dialysator fördern. Die Rückführung des gereinigten Blutes erfolgt auf ähnliche Weise mittels entsprechender Blutpumpen. Im extrakorporalen Blutkreislauf wird das Blut über Schlauchleitungen geführt, die außer mit dem Dialysator auch mit weiteren Vorrichtungen verbunden sein können, z.B. Sensoren zur Erfassung von Eigenschaften des Blutes, des Blutdrucks, etc.

Die Dialysierflüssigkeit wird mit einer oder mehreren Dialysatpumpen aus einem Vorratsbehälter oder einer zentralen Leitung gefördert, einem Eingang des Dialysators zugeführt und an einem entsprechenden Ausgang des Dialysators zur Entsorgung abgenommen. Die Dialysatpumpen können ebenfalls Schlauchrollenpumpen oder Impellerpumpen sein.

Die Plasmapherese ist ein Blutbehandlungsverfahren, bei dem das Patientenblut in das Blutplasma und seine korpuskularen Bestandteile (Zellen) aufgetrennt wird. Das abgetrennte Blutplasma wird gereinigt oder durch eine Substitutionslösung ersetzt und das gereinigte Blutplasma oder die Substitutionslösung dem Patienten zurückgegeben.

Bei der Peritonealdialyse wird die Bauchhöhle eines Patienten über einen durch die Bauchdecke geführten Katheter mit einer Dialyseflüssigkeit befüllt, die ein Konzentrationsgefälle von Blutsubstanzen wie Elektrolyte (beispielsweise Natrium, Kalzium und Magnesium) gegenüber den körpereigenen Flüssigkeiten aufweist. Über das als Membran wirkende Bauchfell (Peritoneum) treten im Körper vorliegende Giftstoffe aus den im Bauchfell verlaufenden Blutgefäßen in die Bauchhöhle über. Nach einigen Stunden wird die sich in der Bauchhöhle des Patienten befindliche, nunmehr mit den aus dem Körper übergetretenen Giftstoffen versetzte Dialyseflüssigkeit ausgetauscht. Durch osmotische Vorgänge kann Wasser aus dem Blut des Patienten über das Bauchfell in die Dialyseflüssigkeit übertreten und den Patienten somit entwässern.

Das Verfahren zur Peritonealdialyse wird in der Regel mit Hilfe von automatischen Peritonealdialysegeräten, wie sie beispielsweise von der Anmelderin vertrieben werden, durchgeführt.

Dialysegeräte, als Beispiel für komplexe medizintechnische Geräte, haben umfangreiche Funktionen und weisen eine Vielzahl von elektrischen und elektronischen Komponenten auf. Um diese Funktionen sowie die elektrischen und elektronischen Komponenten zu steuern, sind medizintechnische Geräte wie Dialysegeräte mit zumindest einem Steuergerät oder einer Steuereinheit ausgerüstet. Diese können eine oder mehrere CPUs (central processing unit) oder Mikrokontroller umfassen, die von Softwareprogrammen gesteuert werden. Im Lichte der Offenbarung der vorliegenden Erfindung ist es nicht wesentlich, ob die beschriebenen Verfahren von einem oder mehreren Steuergeräten durchgeführt werden. Demnach gilt auch eine Vielzahl von Steuergeräten, die einzeln oder im Verbund die beschriebenen Verfahren ausführen, als Steuergerät. Die Softwareprogramme werden in der Regel in einer internen Speichervorrichtung vorgehalten. Weitere Speichervorrichtungen können zum Abspeichern sonstiger Informationen, wie Behandlungsdaten, vorgehalten werden. Das Steuergerät steuert während einer Behandlung eine oder mehrere Pumpen, um einen Blutfluss durch den extrakorporalen Blutkreislauf und die Blutbehandlungsvorrichtung zu bewirken. Außerdem steuert das Steuergerät ein oder mehrere Sensoren an, z.B. Druck- und Flusssensoren, um den Verlauf der Behandlung zu erfassen und ggf. zu regeln, aber auch, um kritische Zustände des Patienten oder der Maschine zu erkennen und die Behandlung entsprechend anzupassen oder abzubrechen bzw. zu unterbrechen.

Während einer Behandlung, die typischerweise zwischen vier und fünf Stunden dauert, wird eine Vielzahl von maschinen- und patientenbezogenen Daten erzeugt, die zu Dokumentations- und Analysezwecken gespeichert werden müssen. Eine Speicherung solcher Daten kann auf einem mit dem Dialysegerät fest oder zeitweise verbundenen Datenträger erfolgen, jedoch müssen die Daten zu einem späteren Zeitpunkt an eine Datenbank übertragen werden, um im Vergleich mit anderen Daten weiter ausgewertet werden zu können. Außerdem muss der begrenzte Speicherplatz von mit dem Dialysegerät fest oder zeitweise verbundenen Datenträgern regelmäßig freigegeben werden, um neue Daten aufzeichnen zu können.

Eine kontinuierliche oder paketweise Übertragung von Daten an die Datenbank während einer Behandlung vermeidet einige der nötigen Arbeitsschritte, die bei einer Übertragung von mit dem Dialysegerät fest oder zeitweise verbundenen Datenträgern erforderlich sind, und reduziert dabei auch mögliche Fehlerquellen. In modernen medizintechnischen Geräten vorgesehene Netzwerk-Schnittstellen ermöglichen den Anschluss an eine entsprechende Netzwerk-Kommunikations-Infrastruktur einer medizinischen Einrichtung.

Moderne medizinische Einrichtungen haben üblicherweise eine komplexe und heterogene Netzwerk-Kommunikations-Infrastruktur, die eine Vielzahl unterschiedlicher Geräte miteinander bzw. mit zentralen Servern und Datenbanken verbindet. Heterogen bedeutet hierbei, dass zum einen eine Netzwerk-Kommunikation von medizintechnischen Geräten untereinander und mit dedizierten Datenbanken und Servern zum Beispiel für die Speicherung und Verarbeitung medizinischer Daten, zum anderen eine Netzwerk-Kommunikation von allgemeinen informationstechnischen Anlagen und Geräten stattfindet. Die Netzwerk-Kommunikations-Infrastruktur umfasst eine Vielzahl von kommunikationsleitenden und -lenkenden Netzwerkknoten, bspw. Router und Switches, die mit Verbindungsleitungen verbunden sind.

Die Netzwerk-Kommunikations-Infrastruktur einer medizinischen Einrichtung und damit verbundene medizintechnische Geräte müssen gesetzliche Vorgaben zur elektrischen Sicherheit einhalten, die u.a. in der Europäischen Norm EN 60601-1 festgelegt sind. Vergleichbare Normen existieren in Ländern außerhalb des Geltungsbereichs der Europäischen Normen.

Die Norm EN 60601-1 definiert unter anderem allgemeine Anforderungen für die Basissicherheit von elektrischen Geräten oder Systemen mit einem Anschluss an ein Stromversorgungsnetz, die zur Diagnose, Behandlung oder Überwachung von Patienten bestimmt sind, und die in direktem körperlichen oder elektrischen Kontakt zum Patienten stehen. Je nach Art des medizintechnischen Geräts können weitere Anforderungen in anderen, ergänzenden Normen definiert sein, u. U. auch derart, dass Anforderungen aus der EN 60601-1 aufgehoben, verändert oder ergänzt werden.

Besonderes Augenmerk wird dabei auf die Sicherheit des Patienten und des medizinischen Personals als Anwender bzw. Bediener der elektrischen Geräte gelegt. Die Sicherheit elektrischer Geräte wird insbesondere durch Isolierungen, Luft- und Kriechstrecken, Beschaffenheit von Komponenten und Erdungen bestimmt. Isolierungen, Luft- und Kriechstrecken werden an einigen Stellen der Beschreibung auch als elektrische oder galvanische Trennung bezeichnet. Die Norm fordert, dass medizinisches Equipment, das in Kontakt mit Patienten kommen kann, mit zwei voneinander unabhängigen Sicherheitsvorkehrungen (MOP, Means of Protection) ausgestattet ist, sodass die elektrische Sicherheit auch beim Ausfall einer der Vorkehrungen weiter gewährleistet ist. Diese Isolationsstufen sind als Anforderungen an die Isolierung des Geräts zum Schutz des Anwenders (Means Of Operator Protection, MOOP) oder des Patienten (Means Of Patient Protection, MOPP) definiert. Aufgrund der erhöhten Risiken für Patienten beim Kontakt mit elektrischem oder elektronischem Equipment sind an MOPP zum Schutz von Patienten höhere Anforderungen zu stellen als an MOOP zum Schutz des Anwenders.

Die EN 60601-1 macht für MOOP und MOPP Vorgaben zur Spannungsfestigkeit der Isolierung und zur Länge der Kriechstrecke zwischen Spannungsführenden Teilen beiderseits der Isolierung. Eine Einstufung als 1 MOOP erfordert eine Spannungsfestigkeit der Isolierung von 1500 VAC und eine Kriechstrecke von 2,5 mm. Bei einer Einstufung als 2 MOOP verdoppeln sich die Anforderungen, es sind also eine Spannungsfestigkeit der Isolierung von 3000 VAC und eine Kriechstrecke von 5 mm gefordert. Die Anforderungen für MOPP sind höher. Hier sind für 1 MOPP eine Spannungsfestigkeit der Isolierung von 1500 VAC und eine Kriechstrecke von 4 mm, und für 2 MOPP eine Spannungsfestigkeit der Isolierung von 4000 VAC und eine Kriechstrecke von 8 mm gefordert.

Die EN 60601-1 stuft Netzwerkverbindungen zwischen medizintechnischen Geräten und einem Kommunikationsnetzwerk einer medizinischen Einrichtung als eine mögliche Gefahrenquelle ein. Beispielsweise kann ein Leckstrom, der aufgrund von Potentialdifferenzen zwischen Erdungsanschlüssen unterschiedlicher miteinander leitend verbundener Netzwerkkomponenten entsteht, eine Gefährdung darstellen. Der Leckstrom kann Messergebnisse von Geräten zur Überwachung von Patienten verfälschen, und dadurch zu Fehldiagnosen und -behandlungen führen, oder sogar zu Fehlfunktionen in Steuervorrichtungen von Geräten zur Behandlung von Patienten führen.

Potentialdifferenzen können z.B. durch eine fehlerhafte Elektroinstallation, Feuchtigkeit in der Elektroinstallation, oder durch Alterung schadhaft gewordene oder von Anfang an schadhafte Kabel zustande kommen.

Die Verwendung von Netzwerkkabeln ohne elektrische Abschirmung stellt keine sichere Lösung des Problems dar, weil auch hiermit keine ausreichende galvanische Isolierung gewährleistet ist. Außerdem weisen solche Netzwerkkabel eine schlechte oder gar keine Unempfindlichkeit gegenüber eingestrahlten Störungen auf, und können selbst Störungen ausstrahlen, was in medizintechnischen Anwendungen unzulässig ist.

Eine Möglichkeit, Netzwerkverbindungen galvanisch derart aufzutrennen und zu isolieren, dass keine Fehlerströme fließen bzw. keine Fremdspannungen anliegen können, sind Netzwerkisolatoren. Netzwerkisolatoren für die Verwendung in einem lokalen Netzwerk (Lokal Area Network, LAN) sind Bauelemente, die jedes an einem Eingang des Netzwerkisolators ankommende Adernpaar einer Netzwerkleitung über einen 1:1-Signalübertrager mit einem entsprechenden Adernpaar an einem Ausgang des Netzwerkisolators verbinden. Der Signalübertrager koppelt dabei die Eingangsseite und die Ausgangsseite magnetisch wie ein Transformator, wobei der Übertrager eine zur Erfüllung der Vorgaben der EN 60601-1 ausreichende Spannungsfestigkeit zwischen der Eingangsseite und der Ausgangsseite sicherstellt.

### Zusammenfassung

Es ist wünschenswert eine alternative Lösung für Schaltung für eine Kommunikationsschnittstelle eines medizintechnischen Gerätes anzugeben, die Mittel zum Schutz von Patienten und/oder Bedienpersonal vor gefährlichen elektrischen Spannungen und/oder Leckströmen aufweist. Es ist außerdem wünschenswert eine Lösung für eine Kommunikationsschnittstelle eines medizintechnischen Gerätes anzugeben, die eine verringerte Empfindlichkeit für elektromagnetische Störungen aufweist.

Erfindungsgemäß wird ein medizintechnisches Gerät gemäß Anspruch 1 bereitgestellt.

Einige der nachfolgend mit Bezug auf ein medizintechnisches Gerät beschriebenen Ausgestaltungen einer Anordnung für eine Kommunikationsschnittstelle erfüllen den Wunsch für einen Schutz von Patienten und/oder Bedienpersonal vor gefährlichen elektrischen Spannungen.

Einige der nachfolgend mit Bezug auf ein medizintechnisches Gerät beschriebenen Ausgestaltungen einer Anordnung für eine Kommunikationsschnittstelle erfüllen den Wunsch nach einer verringerten Empfindlichkeit für elektromagnetische Störungen. Einige der nachfolgend mit Bezug auf ein medizintechnisches Gerät beschriebenen Ausgestaltungen einer Anordnung für eine Kommunikationsschnittstelle erfüllen den Wunsch für einen Schutz von Patienten und/oder Bedienpersonal vor gefährlichen elektrischen Spannungen und den Wunsch nach einer verringerten Empfindlichkeit für elektromagnetische Störungen.

Im Interesse einer besseren Lesbarkeit und Verständlichkeit wird in der vorliegenden Beschreibung an einigen Stellen lediglich eine Wandlung eines elektrischen Signals in ein optisches Signal oder eine Wandlung eines optischen Signals in ein elektrisches Signal beschrieben. Es versteht sich von selbst, dass eine derartige Wandlung bei einer Schnittstelle für eine bidirektionale Kommunikation entsprechend auch in die jeweils andere Richtung erfolgt.

Ein medizintechnisches Gerät mit einer Anordnung für eine

Kommunikationsschnittstelle, welche eine oder mehrere der vorstehend genannten gewünschten Eigenschaften aufweist, umfasst zumindest ein Gehäuse, innerhalb des Gehäuses angeordnete elektrische und/oder elektronische Komponenten und eine Benutzerschnittstelle. Die Benutzerschnittstelle kann ein Display und Bedienelemente umfassen, welche zur Bedienung des medizintechnischen Geräts berührt werden. Ein Beispiel für ein derartiges medizintechnisches Gerät ist ein Dialysegerät, wie es weiter oben ausführlich beschrieben wurde.

Das medizintechnische Gerät weist außerdem eine von außerhalb des Gehäuses zugängliche erste Aufnahmevorrichtung einer ersten Kommunikationsschnittstelle zur Aufnahme eines proximalen Endes eines Kommunikationssignale leitenden Mediums auf. Die von außen zugängliche erste Aufnahmevorrichtung kann beispielsweise eine Buchse eines Netzwerkanschlusses sein, und das Kommunikationssignale leitende Medium ist ein entsprechendes Netzwerkkabel, zum Beispiel ein Kabel für ein lokales Netzwerk (LAN) nach dem IEEE 802.3 Standard (Ethernet) oder eine optische Anschlussleitung für ein faseroptisches Netzwerk, beispielsweise nach einem der Standards der IEC 61754 Familie.

Die erste Kommunikationsschnittstelle ist mit einem oder mehreren Prozessoren kommunikativ verbunden, so dass der eine Prozessor oder die mehreren Prozessoren Daten senden und/oder empfangen können. Die erste Kommunikationsschnittstelle kann auch mit dem einen Prozessor oder den mehreren Prozessoren zusammen als Teil eines auf einem Computerchip integrierten Systems (System-on-Chip, SOC) implementiert sein. Der eine Prozessor oder die mehreren Prozessoren sind außerdem mit flüchtigem und/oder nichtflüchtigem Speicher kommunikativ verbunden, so dass Programminstruktionen von Computerprogrammen sowie Daten ausgelesen, gespeichert und abgearbeitet bzw. verarbeitet werden können.

Ein distales Ende des Kommunikationssignale leitenden Mediums ist zum Anschluss an eine entsprechende zweite Aufnahmevorrichtung vorgesehen, die über ein Kommunikationsnetzwerk mit einer zweiten Kommunikationsschnittstelle verbunden ist. Die zweite Kommunikationsschnittstelle bzw. das Kommunikationsnetzwerk sind dabei nicht Teil des medizintechnischen Gerätes.

Eine Kommunikationsstrecke zwischen dem Kommunikationsnetzwerk und geräteinternen, mit der ersten Kommunikationsschnittstelle kommunikativ in Verbindung stehenden, elektrischen Komponenten des medizintechnischen Geräts weist mindestens zwei Segmente auf. Mindestens eines der Segmente überträgt Kommunikationssignale elektrisch nichtleitend und weist eine elektrische bzw. galvanische Trennung zwischen Eingang und Ausgang bzw. Ausgang und Eingang des Segments mit einer vorgegebenen ersten Spannungsfestigkeit auf.

Eine Kommunikationsverbindung kann dauerhaft oder wahlweise über die Kommunikationsstrecke hergestellt werden, wenn das distale und das proximale Ende des Kommunikationssignale leitenden Mediums mit jeweiligen Aufnahmevorrichtungen verbunden sind.

Bei einer Ausgestaltung des medizintechnischen Geräts ist das Kommunikationssignale elektrisch nichtleitend übertragende Segment beispielsweise eine faseroptische Datenverbindung. Im Falle einer geräteinternen faseroptischen Datenverbindung werden an dem medizintechnischen Gerät ankommende elektrische Datensignale in einem entsprechenden Wandler in optische Datensignale umgesetzt und mindestens dem einen Prozessor oder den mehreren Prozessoren oder weiteren elektrischen oder elektronischen Komponenten innerhalb des medizintechnischen Gerätes zur Weiterverarbeitung oder Weiterleitung zugeführt, gegebenenfalls nach einer Rückwandlung in elektrische Datensignale mittels entsprechender Wandler. Die faseroptische Datenverbindung kann Mono- oder Multimode-Glasfasern umfassen, aber auch lichtleitende Multimode-Kunststofffasern, auch als Plastic Optical Fibre oder POF bekannt. Es können sowohl Stufenindex- als auch Gradientenindexfasern verwendet werden. Eine faseroptische Verbindung kann jedoch auch durch das Kommunikationssignale leitende Medium hergestellt sein, und die Isolation der berührbaren Teile der Kommunikationsschnittstelle des medizintechnischen Gerätes wird durch die faseroptische Verbindung und die erste Aufnahmevorrichtung zur Aufnahme eines optische Kommunikationssignale leitenden Mediums sichergestellt. Die erste Aufnahmevorrichtung kann beispielsweise zur Aufnahme faseroptische Verbindungen nach einem der Standards der IEC 61754 Familie eingerichtet sein.

Die faseroptische Datenverbindung kann für bidirektionalen Betrieb jeweils eine eigene optische Leitung für die Sende- bzw. Empfangsrichtung aufweisen, um einen sogenannten Voll-Duplex-Betrieb zu ermöglichen. Hierbei ist jeweils ein Sender an einem Ende einer optischen Leitung mit einem Empfänger am anderen Ende der optischen Leitung verbunden. Falls das Kommunikationssignale leitende Medium optische Signale überträgt ist die erste Aufnahmevorrichtung entsprechend zur Aufnahme zweier lichtleitender Fasern eingerichtet. Voll-Duplex beschreibt die Möglichkeit, gleichzeitig zu senden und zu empfangen. Alternativ kann für die Sende- bzw. Empfangsrichtung jeweils Licht einer anderen Wellenlänge verwendet werden, das über eine gemeinsame optische Leitung geleitet wird (Wellenlängenduplex bzw. - multiplex). Auch in diesem Fall ist ein Voll-Duplex-Betrieb möglich. Die Ein- bzw. Auskopplung von Licht der jeweiligen Wellenlänge erfolgt in entsprechenden kombinierten Sender-Empfänger-Bausteinen (Transceiver = Transmitter-Receiver). Wenn kein Voll-Duplex-Betrieb nötig ist kann bidirektionale Datenkommunikation auch im sogenannten Halb-Duplex-Betrieb erfolgen, bei dem abwechselnd gesendet bzw. empfangen wird. Hierzu kann eine zeitweise Speicherung der zu sendenden Daten erforderlich sein, bis der jeweilige Sender Daten übertragen kann. Alternativ kann die Erzeugung der zu sendenden Daten über eine entsprechende zeitlich synchronisierte Steuerung erfolgen, wodurch eine senderseitige Speicherung von Daten unnötig sein kann. In der Regel erfolgt eine Steuerung über ein geeignetes Protokoll, das gleichzeitiges Senden ausschließen soll.

Bei einer Ausgestaltung des medizintechnischen Geräts erfolgt die optische Übertragung der Kommunikationssignale in einem Spektrum, das infrarotes und/oder sichtbares Licht umfasst

Bei der erfindungsgemäßen Ausgestaltung des medizintechnischen Geräts ist das mindestens eine Segment der Kommunikationsstrecke, welches Kommunikationssignale elektrisch nichtleitend überträgt, innerhalb des medizintechnischen Geräts angeordnet.

Bei der erfindungsgemäßen Ausgestaltung des medizintechnischen Gerätes, bei der das mindestens eine Segment der Kommunikationsstrecke, welches Kommunikationssignale elektrisch nichtleitend überträgt, innerhalb des medizintechnischen Geräts angeordnet ist, ist an dem oder in dem Gerät eine von einer ersten Spannungsversorgung mit Energie versorgte Schaltung zur Umsetzung elektrischer in optische Signale und/oder optischer in elektrische Signale vorgesehen. Die erste Spannungsversorgung ist dazu eingerichtet, eine galvanische bzw. elektrische Trennung der Schaltung zur Umsetzung elektrischer in optische Signale und/oder optischer in elektrische Signale von anderen, geräteinternen elektrischen und/oder elektronischen Komponenten mit einer Spannungsfestigkeit zu gewährleisten, die mindestens der vorgegebenen ersten Spannungsfestigkeit entspricht. Die die von außerhalb des Gehäuses des medizintechnischen Gerätes zugängliche Aufnahmevorrichtung der ersten Kommunikationsschnittstelle ist dazu eingerichtet, ein elektrische Kommunikationssignale leitendes Medium aufzunehmen. Die elektrisch bzw. galvanisch von anderen geräteinternen elektrischen und/oder elektronischen Komponenten getrennte Schaltung zur Umsetzung wandelt die elektrischen Kommunikationssignale in optische Kommunikationssignale und leitet diese über eine entsprechende Leitung an eine weitere geräteinterne Wandlerschaltung weiter. In der weiteren geräteinternen Wandlerschaltung werden die optischen Signale in elektrische Signale gewandelt und mindestens dem einen Prozessor oder den mehreren Prozessoren oder weiteren elektrischen oder elektronischen Komponenten innerhalb des medizintechnischen Gerätes zugeführt.

Die erste Spannungsversorgung wird erfindungsgemäß von einer außerhalb des medizintechnischen Geräts angeordneten, geräteexternen Energiequelle versorgt, wobei die bereitgestellte Spannung unterhalb einer vorbestimmten als sicher geltenden Maximalspannung liegt.

Im Falle einer Versorgung der ersten Spannungsversorgung mit Energie von einer Spannungsversorgung des medizintechnischen Gerätes kann die erste Spannungsversorgung einen Gleichspannungswandler (DC-DC-Wandler) umfassen, der an eine geeignete Spannungsschiene einer Spannungsversorgung des medizintechnischen Gerätes angeschlossen ist.

Im Falle einer Versorgung der ersten Spannungsversorgung mit Energie von einer geräteexternen Energiequelle kann die externe Spannungsversorgung ein entsprechendes Netzteil umfassen, welches an ein an einem Aufstellort des medizintechnischen Gerätes bereitgestelltes Stromversorgungsnetz angeschlossen ist.

Es ist jedoch auch denkbar, dass an dem Aufstellort des medizintechnischen Gerätes eine als sicher geltende Spannung verfügbar ist, die kleiner ist als die Netzspannung, beispielsweise eine niedrige Gleichspannung von 12 VDC eines Schwesternrufsystems, die dem medizintechnischen Gerät zugeführt ist bzw. durch dieses durchgeschleift wird. In letzterem Fall erfolgt der Anschluss eines Auslösers des Schwesternrufsystems an dem medizintechnischen Gerät. Wenn diese niedrige Gleichspannung mit einer ausreichenden Leistung und/oder niederohmig bereitgestellt ist, kann damit die erste Spannungsversorgung die Schaltung zur Umsetzung elektrischer in optische Signale und/oder optischer in elektrische Signale entweder direkt versorgt werden, oder nach einer entsprechende Reduzierung oder Heraufsetzung auf eine geeignete oder benötigte Spannung, beispielsweise durch einen Linearregler oder einen Schaltwandler. Die Spannungsanpassung benötigt keine besondere elektrische bzw. galvanische Trennung zwischen Eingang und Ausgang und kann daher kostengünstig in bekannter Weise realisiert werden.

Alternativ kann die erste Spannungsversorgung über das Kommunikationssignale leitende Medium mit elektrischer Energie versorgt werden, beispielsweise mittels "Power over Ethernet" (PoE) zusammen mit Daten über eine Ethernetleitung durch das Kommunikationsnetzwerk. Die Einspeisung der Versorgungsspannung in die Ethernetleitung kann dabei an zentraler Stelle in dem Kommunikationsnetzwerk erfolgen, zum Beispiel in einem dazu eingerichteten Router oder Switch. Es ist aber auch denkbar, dass die Einspeisung der Versorgungsspannung in die Ethernetleitung erst in der Nähe des Aufstellortes des medizintechnischen Gerätes erfolgt, beispielsweise direkt an dem Netzwerkanschluss des Kommunikationsnetzwerks. Letztere Lösung kann ohne großen Aufwand leicht nachträglich realisiert werden.

Ein System, welches eine oder mehrere der eingangs genannten gewünschten Eigenschaften aufweist, umfasst das vorstehend beschriebene medizintechnische Gerät, wobei die von außerhalb des Gehäuses des medizintechnischen Gerätes zugängliche erste Aufnahmevorrichtung zur Aufnahme eines proximalen Endes einer optischen Anschlussleitung eines faseroptischen Netzwerks eingerichtet ist. Das System umfasst außerdem die optische Anschlussleitung und eine an dem distalen Ende der optischen Anschlussleitung angeschlossene Vorrichtung zur Wandlung von elektrischen Datensignalen in optische Datensignale und/oder optischen Datensignalen in elektrische Datensignale. Das System nimmt an der an dem distalen Ende der optischen Anschlussleitung angeschlossenen Vorrichtung zur Wandlung von elektrischen Datensignalen in optische Datensignale elektrische Datensignale von der mit dem Kommunikationsnetzwerk verbundenen zweiten Kommunikationsschnittstelle entgegen, wandelt diese in optische Datensignale und leitet sie über die optische Anschlussleitung an die von außerhalb des Gehäuses des medizintechnischen Gerätes zugängliche erste Aufnahmevorrichtung weiter. Mit der ersten Aufnahmevorrichtung des medizintechnischen Gerätes ist ein Wandler kommunikativ verbunden, der die optischen Signale in elektrische Signale wandelt und zur Weiterleitung an den einen Prozessor oder die mehreren Prozessoren sowie ggf. weitere interne elektrische und elektronische Komponenten des medizintechnischen Geräts bereitstellt. Analog empfängt der mit der Aufnahmevorrichtung des medizintechnischen Gerätes kommunikativ verbundene Wandler elektrische Signale von dem einen Prozessor oder den mehreren Prozessoren sowie ggf. den weiteren internen elektrischen und elektronischen Komponenten des medizintechnischen Geräts elektrische Signale und wandelt sie in optische Signale. Die optischen Signale werden über die optische Anschlussleitung an die an dem distalen Ende der optischen Anschlussleitung angeschlossene Vorrichtung zur Wandlung von elektrischen Datensignalen in optische Datensignale und/oder optischen Datensignalen in elektrische Datensignale geleitet, wo sie in elektrische Datensignale gewandelt werden und an die mit dem Kommunikationsnetzwerk verbundene zweite Kommunikationsschnittstelle weitergeleitet werden.

Die Vorrichtung zur Wandlung elektrischer in optische Signale und/oder optischer in elektrische Signale umfasst eine zweite Spannungsversorgung, welche von einer außerhalb des medizintechnischen Geräts liegenden Energiequelle mit Energie versorgt wird. Die Außerhalb des medizintechnischen Geräts liegende Energiequelle kann beispielsweise ein Netzteil sein, das an ein am Aufstellort bereitgestelltes Stromversorgungsnetz angeschlossen ist. Es kann aber auch eine am Aufstellort vorhandene Gleichspannungsversorgung genutzt werden, zum Beispiel die eines Schwesternrufsystems.

Bei einer Ausgestaltung des Systems erfolgt die Energieversorgung der zweiten Spannungsversorgung über die zu dem Kommunikationsnetzwerk gehörende zweite Kommunikationsschnittstelle, beispielsweise mittels PoE über das Kommunikationssignale leitende Medium, welches zwischen der zu dem Kommunikationsnetzwerk gehörenden zweiten Kommunikationsschnittstelle und der Vorrichtung zur Wandlung von elektrischen Datensignalen in optische Datensignale und/oder optischen Datensignalen in elektrische Datensignale angeordnet ist.

Die PoE-Energieeinspeisung kann, wie in der weiter oben beschriebenen Ausgestaltung, an einer zentralen Stelle in dem Kommunikationsnetzwerk erfolgen, zum Beispiel in einem dazu eingerichteten Router oder Switch. Es ist aber auch denkbar, dass die Einspeisung der Versorgungsspannung in die Ethernetleitung erst in der Nähe des Aufstellortes des medizintechnischen Gerätes erfolgt, beispielsweise direkt an dem Netzwerkanschluss des Kommunikationsnetzwerks. Letztere Lösung kann, wie zuvor bereits erwähnt, ohne großen Aufwand leicht nachträglich realisiert werden.

### Kurze Beschreibung der Zeichnung

Im Folgenden wird die Schaltung für eine Kommunikationsschnittstelle eines medizintechnischen Gerätes exemplarisch mit Bezug auf die Figuren der Zeichnung beschrieben. In der Zeichnung zeigt
- Fig. 1: ein medizintechnisches Gerät mit einer ersten bekannten Ausführung einer Kommunikationsschnittstelle,
- Fig. 2: ein medizintechnisches Gerät mit einer zweiten bekannten Ausführung einer Kommunikationsschnittstelle,
- Fig. 3: ein erstes Ausführungsbeispiel eines medizintechnischen Gerätes mit einer für den Schutz von Patienten und Bediener eingerichteten Kommunikationsschnittstelle,
- Fig. 4: ein zweites Ausführungsbeispiel eines medizintechnischen Gerätes mit einer für den Schutz von Patienten und Bediener eingerichteten Kommunikationsschnittstelle,
- Fig. 5: ein drittes Ausführungsbeispiel eines medizintechnischen Gerätes mit einer für den Schutz von Patienten und Bediener eingerichteten Kommunikationsschnittstelle,
- Fig. 6: ein erstes Ausführungsbeispiel eines Systems mit einem medizintechnischen Gerät mit einer für den Schutz von Patienten und Bediener eingerichteten Kommunikationsschnittstelle,
- Fig. 7: ein zweites Ausführungsbeispiel eines Systems mit einem medizintechnischen Gerät mit einer für den Schutz von Patienten und Bediener eingerichteten Kommunikationsschnittstelle, und
- Fig. 8: ein viertes Ausführungsbeispiel eines medizintechnischen Gerätes mit einer für den Schutz von Patienten und Bediener eingerichteten Kommunikationsschnittstelle.

In den Figuren sind gleiche oder ähnliche Elemente mit denselben Referenzbezeichnungen versehen.

### Beschreibung der Ausführungsformen

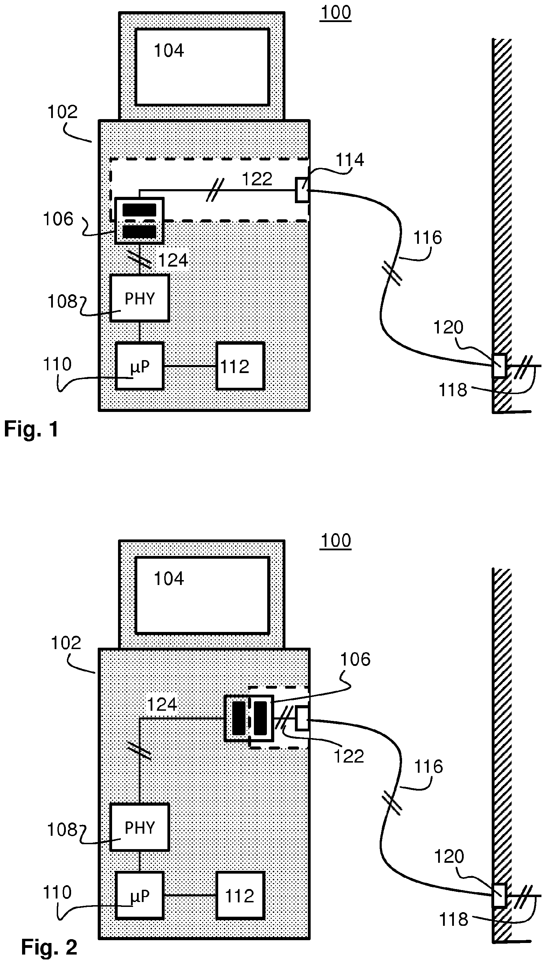

Figur 1 zeigt ein medizintechnisches Gerät 100 mit einer ersten bekannten Ausführung einer Kommunikationsschnittstelle. Das medizintechnische Gerät 100 umfasst ein Gehäuse 102, innerhalb dessen elektrische und/oder elektronische Komponenten angeordnet sind. Die elektrischen und/oder elektronischen Komponenten sind in der Figur durch einen Datenübertrager 106, eine Schaltung 108 (PHY), welche einen Übergabepunkt zwischen einem logischen Teil der Kommunikationsschnittstelle und der physikalischen Übertragung implementiert, einen Mikroprozessor 110 (µP) und einen Speicher 112 repräsentiert. Weitere elektrische und/oder elektronische Komponenten, bspw. Pumpen, Aktuatoren, Steuerschaltungen und dergleichen, sind aus Gründen der Übersichtlichkeit in dieser und den folgenden Figuren nicht gezeigt. Das medizintechnische Gerät 100 weist außerdem eine Benutzerschnittstelle 104 auf, in der Figur durch einen Bildschirm repräsentiert. Die Benutzerschnittstelle kann weitere Elemente umfassen, bspw. Schalter, Knöpfe, Hebel, Regler, etc., die aus Gründen der Übersichtlichkeit in dieser und den folgenden Figuren nicht dargestellt sind. Das medizintechnische Gerät 100 weist darüber hinaus eine Aufnahmevorrichtung 114 für ein Netzwerkkabel 116, bspw. ein LAN-Kabel auf, welches das medizintechnische Gerät 100 über ein Kommunikationsnetzwerk 118 mit einer nicht zu dem medizintechnischen Gerät gehörenden zweiten Kommunikationsschnittstelle (nicht in der Figur gezeigt) verbindet. Der Anschluss des Netzwerkkabels 116 an das Kommunikationsnetzwerk 118 kann über eine zweite Aufnahmevorrichtung 120 erfolgen, bspw. eine Netzwerkanschlussdose.

Das Netzwerkkabel 116 stellt eine elektrisch leitende Verbindung zwischen geräteinternen elektrischen und elektronischen Komponenten sowie mit dem Kommunikationsnetzwerk verbundenen elektrischen und elektronischen Geräten und Vorrichtungen (nicht in der Figur gezeigt) her. Datenübertrager 106 bewirkt lediglich eine funktionale Isolierung der Netzwerkschnittstelle, bspw. um Gleichtaktstörungen von der ersten Kommunikationsschnittstelle fernzuhalten. Zudem liegt der Datenübertrager 106 weit innerhalb des medizintechnischen Gerätes 100. Verbindungsleitung 122, die die Aufnahmevorrichtung 114 für das Netzwerkkabel 116 geräteintern mit dem Datenübertrager 106 verbindet ist elektrisch mit dem Netzwerkkabel 116 verbunden und führt dieselbe Spannung wie letzteres. Bei Schäden der Isolation können dadurch Teile des medizintechnischen Gerätes 100 spannungsführend werden und einen mit dem medizintechnischen Gerät 100 verbundenen Patienten oder einen Bediener gefährden. Der von auf dem Netzwerkkabel 116 anliegenden Gleichspannungsstörungen gefährdete Bereich ist in der Figur durch den gestrichelt umrandeten, nicht Grau gefärbten Bereich angedeutet. Außerdem können über das Netzwerkkabel 116 empfangene elektromagnetische Störungen durch die Verbindung mit der Verbindungsleitung 122 in das Innere des Gehäuses 102 medizintechnischen Gerätes 100 geleitet werden und dort unter anderem Mess- und/oder Steuerschaltungen in unerwünschter Weise beeinflussen.

Figur 2 zeigt ein medizintechnisches Gerät 100 mit einer zweiten bekannten Ausführung einer Kommunikationsschnittstelle. Das in dieser Figur gezeigt medizintechnische Gerät 100 entspricht weitgehend dem mit Bezug auf Figur 1 beschriebenen medizintechnischen Gerät 100. Im Unterschied zu dem mit Bezug auf Figur 1 beschriebenen medizintechnischen Gerät 100 ist der Datenübertrager 106 sehr nahe an der Aufnahmevorrichtung 114 für das Netzwerkkabel 116 platziert, wodurch die Verbindungsleitung 122 sehr kurz wird und ggf. ganz entfallen kann. Da der Datenübertrager 106 jedoch keine oder nur eine sehr geringe Filterwirkung gegenüber in die Netzwerkleitung 116 eingekoppelte elektromagnetischen Störungen bewirkt, können diese sich über die Verbindungsleitung 124 im Innern des Gehäuses 102 des medizintechnischen Gerätes 100 ausbreiten und dort unerwünschte Wirkungen zeigen. Außerdem stellt die lediglich funktionale Isolierung des Datenübertragers 106 genauso wie bei der in Figur 1 gezeigten Ausführung keinen ausreichenden Schutz von Patient oder Bediener gegen Überspannungen dar. Wie in Figur 1 ist der von auf dem Netzwerkkabel 116 anliegenden Gleichspannungsstörungen gefährdete Bereich in der Figur durch den gestrichelt umrandeten, nicht Grau gefärbten Bereich angedeutet.

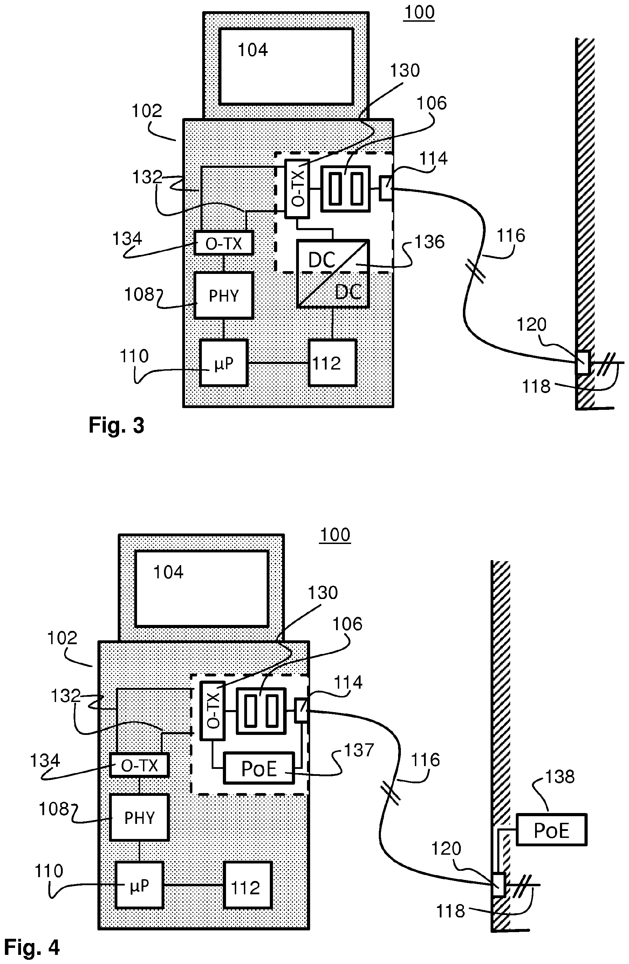

Figur 3 zeigt ein erstes Ausführungsbeispiel eines medizintechnischen Gerätes mit einer für den Schutz von Patienten und Bediener eingerichteten Kommunikationsschnittstelle. Das medizintechnische Gerät 100 umfasst ein Gehäuse 102, innerhalb dessen elektrische und/oder elektronische Komponenten angeordnet sind. Die elektrischen und/oder elektronischen Komponenten sind in der Figur durch eine Schaltung 108 (PHY), welche einen Übergabepunkt zwischen einem logischen Teil der Kommunikationsschnittstelle und der physikalischen Übertragung implementiert, einen Mikroprozessor 110 (µP) und einen Speicher 112 repräsentiert. Weitere elektrische und/oder elektronische Komponenten, bspw. Pumpen, Aktuatoren, Steuerschaltungen und dergleichen, sind aus Gründen der Übersichtlichkeit in dieser und den folgenden Figuren nicht gezeigt. Das medizintechnische Gerät 100 weist außerdem eine Benutzerschnittstelle 104 auf, in der Figur durch einen Bildschirm repräsentiert, der auch berührungssensitiv sein kann. Die Benutzerschnittstelle kann weitere Elemente umfassen, bspw. Schalter, Knöpfe, Hebel, Regler, etc., die aus Gründen der Übersichtlichkeit in dieser und den folgenden Figuren nicht dargestellt sind. Das medizintechnische Gerät 100 weist darüber hinaus eine Aufnahmevorrichtung 114 für ein Netzwerkkabel 116, bspw. ein LAN-Kabel auf, welches das medizintechnische Gerät 100 über ein Kommunikationsnetzwerk 118 mit einer nicht zu dem medizintechnischen Gerät gehörenden zweiten Kommunikationsschnittstelle (nicht in der Figur gezeigt) verbindet.

Das Netzwerkkabel 116 stellt eine elektrisch leitende Verbindung zwischen der Aufnahmevorrichtung 114 für ein Netzwerkkabel 116 und mit dem Kommunikationsnetzwerk verbundenen elektrischen und elektronischen Geräten und Vorrichtungen (nicht in der Figur gezeigt) her. Der mit der Aufnahmevorrichtung 114 verbundene Datenübertrager 106 bewirkt lediglich eine funktionale Isolierung der Netzwerkschnittstelle, bspw. um Gleichtaktstörungen von der ersten Kommunikationsschnittstelle fernzuhalten, und kann, abhängig von der Art und Anordnung der weiteren Schaltungsteile, weggelassen werden. Über das Netzwerkkabel 116 und die Aufnahmevorrichtung 114 an das medizintechnische Gerät geleitete elektrische Signale werden in einer ersten Schaltung (O-TX) 130 zur Umsetzung von elektrischen in optische Signale umgesetzt und über ein optische Signale leitendes Medium 132 an eine zweite Schaltung (O-TX) 134 zur Umsetzung von optischen in elektrische Signale geleitet, dort umgesetzt und über weitere Schnittstellenkomponenten (PHY) 108 an den Mikroprozessor 110 weitergeleitet. Entsprechend werden von dem Mikroprozessor 110 gesendete Signale über die weiteren Schnittstellenkomponenten 108 an zweite Schaltung (O-TX) 134 zur Umsetzung von elektrischen in optische Signale geleitet, über das optische Signale leitende Medium 132 an die erste Schaltung (O-TX) 130 zur Umsetzung von optischen in elektrische Signale geleitet, dort umgesetzt und über die Aufnahmevorrichtung 114 an das Netzwerkkabel 116 geleitet. Das optische Signale leitende Medium 132 kann ein für beide Kommunikationsrichtungen gemeinsam genutztes Medium sein oder zwei getrennte Medien umfassen, von denen jedes jeweils für Signale einer Kommunikationsrichtung genutzt wird.

Eine Stromversorgung der ersten Schaltung 130 erfolgt über einen von einem geräteinternen Netzteil mit Energie versorgten Gleichspannungswandler (DC/DC) 136, der die elektrische bzw. galvanische Trennung mit der vorgegebenen ersten Spannungsfestigkeit sicherstellt. Gleichspannungswandler 136 kann, je nach Bedarf, zudem eine oder mehrere weitere Versorgungsspannungen bereitstellen. Die erste Schaltung 130, der Gleichspannungswandler 136 sowie ggf. der Datenübertrager 106 sind vorzugsweise räumlich nah beieinander angeordnet und von anderen in dem Gehäuse 102 befindlichen elektrischen Komponenten soweit beabstandet angeordnet oder durch andere konstruktive Maßnahmen von diesen getrennt, bspw. durch nichtleitende und/oder abschirmende Kapselung, so dass die elektrische bzw. galvanische Trennung mit der vorgegebenen ersten Spannungsfestigkeit sichergestellt ist.

Bei dem vorstehend beschriebenen Ausführungsbeispiel können über das Kommunikationsnetzwerk bzw. damit verbundene elektrische Geräte und Einrichtungen gefährliche Spannungen nur bis an die durch ausreichenden Abstand oder andere konstruktive Maßnahmen, bspw. eine nichtleitende und/oder abschirmende Kapselung, separierte Eingangsschaltung gelangen und nicht auf andere interne Bauteile des medizintechnischen Geräts wirken. Auch die Ausbreitung von über das Netzwerkkabel 116 eingekoppelten Störungen im Innern des medizintechnischen Geräts wird verringert oder verhindert. Der von auf dem Netzwerkkabel 116 anliegenden gefährlichen Spannungen und Störungen gefährdete Bereich ist in der Figur durch den gestrichelten, nicht Grau gefärbten Bereich angedeutet.

Figur 4 zeigt ein zweites Ausführungsbeispiel eines medizintechnischen Gerätes mit einer für den Schutz von Patienten und Bediener eingerichteten Kommunikationsschnittstelle. Die Ausführung gleicht in den meisten Elementen der mit Bezug auf Figur 3 beschriebenen Ausführung. Im Unterschied zu der zuvor mit Bezug auf Figur 3 beschriebenen Ausführung erfolgt bei dieser Ausführung die Versorgung der ersten Schaltung 130 mit elektrischer Energie von einer externen Energiequelle, wobei das Netzwerkkabel 116 neben den Kommunikationssignalen auch die elektrische Energie leitet. Eine bekannte Ausführung der Versorgung von elektrischen und elektronischen Bauteilen über ein Netzwerkkabel ist unter dem Begriff PoE ("Power over Ethernet") bekannt. Entsprechend ist der Gleichspannungswandler 136 aus Figur 3 in der vorliegenden Figur 4 durch eine Schaltung 137 zur Trennung von Energie und Daten ersetzt, die ihrerseits einen oder mehrere Gleichspannungswandler umfassen kann.

Die erste Schaltung 130, die Schaltung 137 zur Trennung von Energie und Daten sowie ggf. der Datenübertrager 106 sind vorzugsweise räumlich nah beieinander angeordnet und von anderen in dem Gehäuse 102 befindlichen elektrischen Komponenten soweit beabstandet angeordnet oder durch andere konstruktive Maßnahmen von diesen getrennt, bspw. durch nichtleitende und/oder abschirmende Kapselung, so dass die elektrische bzw. galvanische Trennung mit der vorgegebenen ersten Spannungsfestigkeit sichergestellt ist.

Die Versorgung von Schaltungen mit elektrischer Energie über ein Netzwerkkabel erfordert, dass an irgendeiner Stelle die elektrische Energie in das Netzwerkkabel eingespeist wird. Solche Schaltungen sind für PoE kommerziell erhältlich und werden auch als PoE-Injektoren bezeichnet. In der Figur ist ein PoE-Injektor 138 mit der zweiten Aufnahmevorrichtung 120 verbunden und speist an dieser Stelle elektrische Energie zur Versorgung der ersten Schaltung 130 in das Netzwerkkabel ein. Alternativ kann auch eine PoE-Einspeisung in einer zentralen Netzwerkkomponente erfolgen, bspw. einem entsprechend eingerichteten Router oder Switch (nicht in der Figur gezeigt).

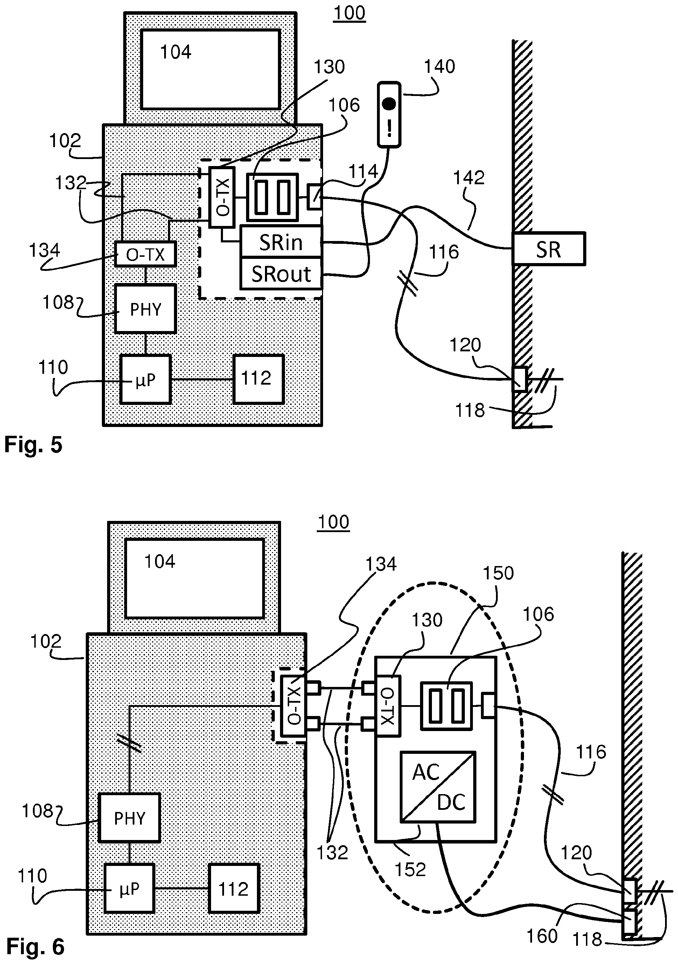

Figur 5 zeigt ein drittes Ausführungsbeispiel eines medizintechnischen Gerätes mit einer für den Schutz von Patienten und Bediener eingerichteten Kommunikationsschnittstelle. Die Ausführung gleicht in den meisten Elementen der mit Bezug auf Figur 4 beschriebenen Ausführung. Im Unterschied zu der zuvor mit Bezug auf Figur 4 beschriebenen Ausführung erfolgt bei dieser Ausführung die Versorgung der ersten Schaltung 130 mit elektrischer Energie von einer externen Energiequelle nicht über das Netzwerkkabel 116, sondern über eine andere externe Energiequelle, die eine als sicher eingestufte, niedrige elektrische Spannung mit ausreichender elektrischer Trennung zum Schutz von Patienten und Bedienern bereitstellt, bspw. ein Schwesternrufsystem.

Üblicherweise wird ein Auslöseschalter 140 für das Schwesternrufsystem an einen entsprechenden Anschluss SR an einem Behandlungsplatz mittels einer Steckverbindung angeschlossen. Der Auslöseschalter 140 kann auch noch weitere Bedienelemente umfassen, bspw. zur Bedienung von in einem Raum befindlichen elektrischen Anlagen, etwa zur Beleuchtung oder Jalousiensteuerung oder dergleichen (nicht in der Figur gezeigt). Der raumseitige Anschluss SR kann bspw. in oder an einer Wand des Raumes, oder an einem sogenannten Anschlusspanel bzw. einer Anschlusssäule angeordnet sein, wobei letztere eine Vielzahl von an dem Behandlungsplatz erforderliche Anschlüsse für elektrische Energie, Datenverbindungen, Sauerstoff- und/oder Druckluftversorgung und dergleichen bereitstellt. In Figur 5 ist der Auslöseschalter 140 an einem entsprechenden Anschluss SRout angeschlossen, der in oder an dem medizintechnischen Gerät 100 angeordnet ist. Das medizintechnische Gerät 100 weist einen Anschluss SRin auf, der mit dem raumseitigen Anschluss SR des Schwesternrufsystems über eine Anschlussleitung 142 verbunden ist. Das Schwesternrufsystem stellt eine Versorgungsspannung für die erste Schaltung 130 bereit, die in dem medizintechnischen Gerät 100 ausgekoppelt wird. Die für den Auslöseschalter 140 benötigten Anschlüsse werden von dem Anschluss SRin zu dem Anschluss SRout durchgeschleift.

Die erste Schaltung 130, Anschlüsse SRin, SRout, sowie ggf. der Datenübertrager 106 sind vorzugsweise räumlich nah beieinander angeordnet und von anderen in dem Gehäuse 102 befindlichen elektrischen Komponenten soweit beabstandet angeordnet oder durch andere konstruktive Maßnahmen von diesen getrennt, bspw. durch nichtleitende und/oder abschirmende Kapselung, so dass die elektrische bzw. galvanische Trennung mit der vorgegebenen ersten Spannungsfestigkeit sichergestellt ist.

Figur 6 zeigt ein erstes Ausführungsbeispiel eines Systems mit einem medizintechnischen Gerät mit einer für den Schutz von Patienten und Bediener eingerichteten Kommunikationsschnittstelle. Bei dieser Ausführung des Systems ist die von außerhalb des Gehäuses 102 des medizintechnischen Gerätes 100 zugängliche erste Aufnahmevorrichtung zur Aufnahme eines proximalen Endes eines optische Signale leitenden Mediums 132. Die erste Aufnahmevorrichtung kann eine entsprechend eingerichtete zweite Schaltung (O-TX) 134 zur Umsetzung von optischen in elektrische Signale umfassen, die ankommende optische Signale umsetzt und über geräteinterne Leitungen sowie Schnittstellenkomponenten (PHY) 108 an den Mikroprozessor 110 weiterleitet. Zur Kommunikation in der Gegenrichtung setzt die zweite Schaltung 134 von dem Mikroprozessor 110 kommende, über geräteinterne Leitungen sowie Schnittstellenkomponenten (PHY) 108 an die zweite Schaltung 134 gelangende elektrische Signale in optische Signale um und stellt sie außerhalb des medizintechnischen Geräts 100 bereit.

Das System umfasst bei dieser Ausführung eine außerhalb des medizintechnischen Geräts 100 liegende Vorrichtung 150 mit einer ersten Schaltung 130 zur Umsetzung von elektrischen in optische Signale bzw. zur Umsetzung von optischen in elektrische Signale, die über ein optische Signale leitendes Medium 132 mit der zweiten Schaltung (O-TX) 134 zur Umsetzung von optischen in elektrische Signale bzw. zur Umsetzung von elektrischen in optische Signale verbunden ist. Das optische Signale leitende Medium 132 kann ein für beide Kommunikationsrichtungen gemeinsam genutztes Medium sein oder zwei getrennte Medien umfassen, von denen jedes jeweils für Signale einer Kommunikationsrichtung genutzt wird.

Die erste Schaltung 130 setzt von dem medizintechnischen Gerät 100 kommende optische Signale in elektrische Signale um und leitet sie an das Kommunikationsnetzwerk 118 weiter. Zu diesem Zweck kann die erste Schaltung 130 über eine Aufnahmevorrichtung 114 für ein Netzwerkkabel 116 und das Netzwerkkabel 116 mit dem Kommunikationsnetzwerk 118 verbunden sein. Netzwerkkabel 116 stellt eine elektrisch leitende Verbindung zwischen der Aufnahmevorrichtung 114 für ein Netzwerkkabel 116 und mit dem Kommunikationsnetzwerk verbundenen elektrischen und elektronischen Geräten und Vorrichtungen (nicht in der Figur gezeigt) her. Der mit der Aufnahmevorrichtung 114 verbundene Datenübertrager 106 bewirkt lediglich eine funktionale Isolierung der Netzwerkschnittstelle, bspw. um Gleichtaktstörungen von der ersten Kommunikationsschnittstelle fernzuhalten, und kann, abhängig von der Art und Anordnung der weiteren Schaltungsteile, weggelassen werden.

Die erste Schaltung 130 und damit elektrisch leitend verbundene Schaltungsteile sind Teil einer in einem Gehäuse angeordneten Vorrichtung 150. Das Gehäuse stellt den erforderlichen Schutz vor Berührung spannungsführender Teile sicher. Eine Stromversorgung der ersten Schaltung 130 erfolgt über ein von dem medizintechnischen Gerät 100 unabhängiges Netzteil 152, das eine elektrische bzw. galvanische Trennung mit der vorgegebenen ersten Spannungsfestigkeit sicherstellt. Das Netzteil 152 kann in dem Gehäuse angeordnet sein, oder als externes Steckernetzteil ausgeführt sein. Das Netzteil 152 wird von einem lokal bereitgestellten Netzspannungsanschluss 160 mit elektrischer Energie versorgt. Bei einer nicht in der Figur gezeigten Ausführung erfolgt die Versorgung der ersten Schaltung 130 und der weiteren Schaltungsteile mit elektrischer Energie über das Netzwerkkabel 116, bspw. mittels PoE, wie weiter oben mit Bezug auf Figur 4 beschrieben.

Die erste Schaltung und damit unmittelbar verbundene Komponenten, in der Figur angedeutet durch die gestrichelte Linie, können nahe an dem medizintechnischen Gerät 100 oder nahe an raumseitigen Netzwerk- und Netzspannungsanschlüssen angeordnet sein, abhängig von der jeweiligen Länge des Netzwerkkabels 116 und des optische Signale leitenden Mediums 132.

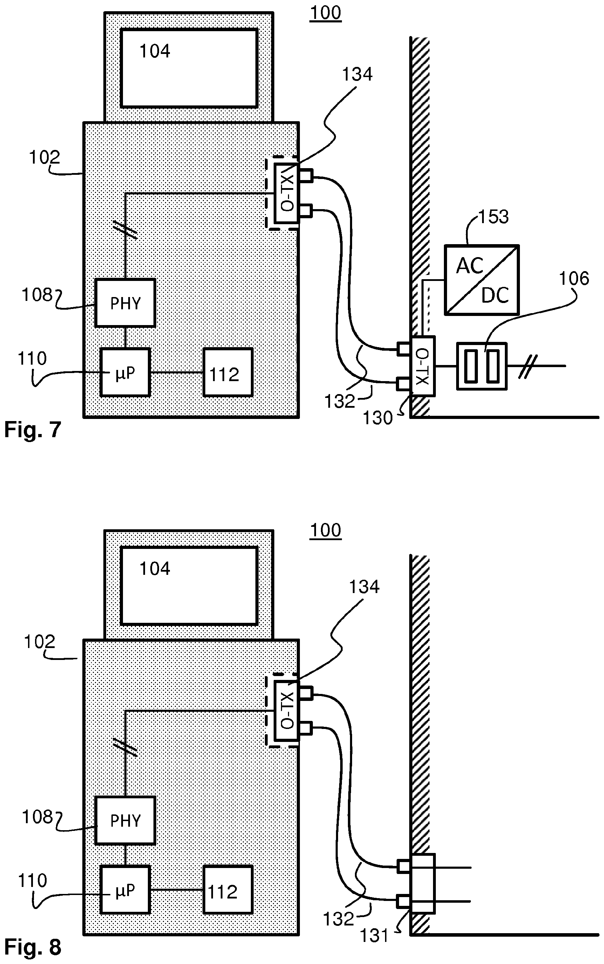

Figur 7 zeigt ein zweites Ausführungsbeispiel eines Systems mit einem medizintechnischen Gerät mit einer für den Schutz von Patienten und Bediener eingerichteten Kommunikationsschnittstelle. Das System entspricht weitgehend dem mit Bezug auf Figur 6 beschriebenen System. Im Unterschied dazu sind die erste Schaltung 130 und damit elektrisch leitend verbundene Schaltungsteile nicht in einem separaten Gehäuse angeordnet, sondern fester Bestandteil einer raumseitigen Installation. Die Stromversorgung der ersten Schaltung 130 erfolgt über ein in der Installation vorgesehenes Netzteil 153, das installationsseitig eine elektrische bzw. galvanische Trennung mit der vorgegebenen ersten Spannungsfestigkeit sicherstellt.

Figur 8 zeigt ein viertes Ausführungsbeispiel eines medizintechnischen Gerätes 100 mit einer für den Schutz von Patienten und Bediener eingerichteten Kommunikationsschnittstelle. Das medizintechnische Gerät 100 entspricht dem mit Bezug auf Figur 6 beschriebenen medizintechnischen Gerät. In diesem Ausführungsbeispiel ist raumseitig ein Anschluss 131 an ein optisches Kommunikationsnetzwerk vorgesehen. Ein optische Signale leitendes Medium 132 verbindet die zweite Schaltung (O-TX) 134 des medizintechnischen Geräts 100 mit dem raumseitigen Anschluss 131.

## Patentansprüche

1. Medizintechnisches Gerät (100) mit einem Gehäuse (102), innerhalb des
Gehäuses (102) angeordneten elektrischen und/oder elektronischen Komponenten, und einer Benutzerschnittstelle (104),
wobei das medizintechnische Gerät (100) außerdem eine von außerhalb des Gehäuses (102) zugängliche erste Aufnahmevorrichtung (114) einer ersten Kommunikationsschnittstelle (106-108) zur Aufnahme eines proximalen Endes eines Kommunikationssignale leitenden Mediums (116) aufweist,
wobei ein distales Ende des Kommunikationssignale leitenden Mediums (116) zum Anschluss an eine zweite Aufnahmevorrichtung (120) vorgesehen ist, die über ein nicht zu dem medizintechnischen Gerät (100) gehörendes Kommunikationsnetzwerk (118) mit einer nicht zu dem medizintechnischen Gerät (100) gehörenden zweiten Kommunikationsschnittstelle verbunden ist,
wobei eine Kommunikationsstrecke zwischen dem Kommunikationsnetzwerk (118) und geräteinternen, mit der ersten Kommunikationsschnittstelle kommunikativ in Verbindung stehenden, elektrischen Komponenten des medizintechnischen Geräts (100) mindestens zwei Segmente aufweist,
und wobei mindestens eines der Segmente Kommunikationssignale elektrisch nichtleitend überträgt und eine elektrische Trennung zwischen Eingang und Ausgang bzw. Ausgang und Eingang des Segments mit einer vorgegebenen ersten Spannungsfestigkeit aufweist,
wobei das mindestens eine Segment der Kommunikationsstrecke, welches Kommunikationssignale elektrisch nichtleitend überträgt, innerhalb des Gehäuses (102) des medizintechnischen Geräts (100) angeordnet ist und wobei an dem oder in dem Gerät (100) eine erste Schaltung (130) zur Umsetzung elektrischer in optische Signale und/oder optischer in elektrische Signale vorgesehen ist, **dadurch gekennzeichnet, dass** die erste Schaltung (130) von einer außerhalb des medizintechnischen Geräts angeordneten Spannungsversorgung (138; SR) mit elektrischer Energie versorgt wird, wobei die bereitgestellte Spannung unterhalb einer vorbestimmten, als sicher geltenden Maximalspannung liegt.

2. Medizintechnisches Gerät (100) nach Anspruch 1, wobei das mindestens eine Segment der Kommunikationsstrecke, das Kommunikationssignale elektrisch nichtleitend überträgt, die Kommunikationssignale optisch überträgt.

3. Medizintechnisches Gerät (100) nach Anspruch 2, wobei die optische Übertragung der Kommunikationssignale in einem Spektrum erfolgt, das infrarotes und/oder sichtbares Licht umfasst.

4. Medizintechnisches Gerät (100) nach Anspruch 1, wobei an dem oder in dem medizintechnischen Gerät (100) eine von einer ersten Spannungsversorgung (136) mit Energie versorgte erste Schaltung (130) zur Umsetzung elektrischer in optische Signale und/oder optischer in elektrische Signale vorgesehen ist, wobei die erste Spannungsversorgung (136) eine elektrische Isolierung der ersten Schaltung (130) zur Umsetzung elektrischer in optische Signale und/oder optischer in elektrische Signale von anderen, geräteinternen elektrischen und/oder elektronischen Komponenten mit einer Spannungsfestigkeit gewährleistet, die mindestens der vorgegebenen ersten Spannungsfestigkeit entspricht.

5. Medizintechnisches Gerät (100) nach Anspruch 1, wobei an dem oder in dem Gerät (100) eine erste Schaltung (130) zur Umsetzung elektrischer in optische Signale und/oder optischer in elektrische Signale vorgesehen ist, welche über das Kommunikationssignale leitende Medium (116) mit elektrischer Energie versorgt wird, wobei eine hierüber bereitgestellte Versorgungsspannung unterhalb einer vorbestimmten, als sicher geltenden Maximalspannung liegt.

6. Medizintechnisches Gerät (100) nach Anspruch 1 oder 2, wobei die erste Aufnahmevorrichtung zur Aufnahme eines optische Kommunikationssignale leitenden Mediums eingerichtet ist.

7. System (200) mit einem medizintechnischen Gerät (100) nach Anspruch 1, wobei die von außerhalb des Gehäuses (102) des medizintechnischen Gerätes (100) zugängliche erste Aufnahmevorrichtung (134) zur Aufnahme eines proximalen Endes eines optische Signale leitenden Mediums (132) eingerichtet ist,
wobei das System (200) außerdem das optische Signale leitende Medium (132) umfasst,
wobei das System (200) an dem distalen Ende des optische Signale leitenden Mediums (132) eine Vorrichtung (150) zur Wandlung elektrischer in optische Signale und/oder optischer in elektrische Signale mit einer zweiten Spannungsversorgung (152) umfasst, und
wobei die zweite Spannungsversorgung von einer außerhalb des medizintechnischen Gerätes (100) liegenden Energiequelle mit Energie versorgt wird.

8. System (200) nach Anspruch 7 wobei die zweite Spannungsversorgung (152) über ein zwischen der zu dem Kommunikationsnetzwerk (118) gehörenden zweiten Kommunikationsschnittstelle und der Vorrichtung zur Wandlung von elektrischen Datensignalen in optische Datensignale und/oder optischen Datensignalen in elektrische Datensignale angeordnetes Kommunikationssignale leitendes Medium mit elektrischer Energie versorgt wird.

## Claims

1. Medical device (100) having a housing (102), having electrical and/or electronic components arranged inside the housing (102), and having a user interface (104),
wherein the medical device (100) also has a first receiving apparatus (114) of a first communication interface (106-108), which receiving apparatus is accessible from outside the housing (102) and is intended to receive a proximal end of a medium (116) conducting communication signals,
wherein a distal end of the medium (116) conducting communication signals is provided for connection to a second receiving apparatus (120) which is connected to a second communication interface that does not belong to the medical device (100) via a communication network (118) that does not belong to the medical device (100),
wherein a communication path between the communication network (118) and electrical components of the medical device (100) which are inside the device and are communicatively connected to the first communication interface has at least two segments, and wherein at least one of the segments transmits communication signals in an electrically non-conductive manner and has electrical isolation between the input and the output or the output and the input of the segment with a predefined first dielectric strength,
wherein the at least one segment of the communication path which transmits communication signals in an electrically non-conductive manner is arranged inside the housing (102) of the medical device (100), and wherein a first circuit (130) for converting electrical signals into optical signals and/or for converting optical signals into electrical signals is provided on or in the device (100), **characterized in that** the first circuit (130) is supplied with electrical energy by a voltage supply (138; SR) arranged outside the medical device, wherein the voltage provided is below a predetermined maximum voltage which is considered to be safe.

2. Medical device (100) according to Claim 1, wherein the at least one segment of the communication path which transmits communication signals in an electrically non-conductive manner transmits the communication signals optically.

3. Medical device (100) according to Claim 2, wherein the communication signals are transmitted optically in a spectrum which comprises infrared and/or visible light.

4. Medical device (100) according to Claim 1, wherein a first circuit (130) which is supplied with energy by a first voltage supply (136) and is intended to convert electrical signals into optical signals and/or to convert optical signals into electrical signals is provided on or in the medical device (100), wherein the first voltage supply (136) ensures electrical insulation of the first circuit (130) for converting electrical signals into optical signals and/or for converting optical signals into electrical signals from other electrical and/or electronic components inside the device with a dielectric strength corresponding at least to the predefined first dielectric strength.

5. Medical device (100) according to Claim 1, wherein a first circuit (130) for converting electrical signals into optical signals and/or for converting optical signals into electrical signals is provided on or in the device (100) and is supplied with electrical energy via the medium (116) conducting communication signals, wherein a supply voltage provided hereby is below a predetermined maximum voltage which is considered to be safe.

6. Medical device (100) according to Claim 1 or 2, wherein the first receiving apparatus is configured to receive a medium conducting optical communication signals.

7. System (200) having a medical device (100) according to Claim 1, wherein the first receiving apparatus (134) which is accessible from outside the housing (102) of the medical device (100) is configured to receive a proximal end of a medium (132) conducting optical signals,
wherein the system (200) also comprises the medium (132) conducting optical signals,
wherein the system (200) comprises an apparatus (150) for converting electrical signals into optical signals and/or for converting optical signals into electrical signals with a second voltage supply (152) at the distal end of the medium (132) conducting optical signals, and
wherein the second voltage supply is supplied with energy by an energy source outside the medical device (100) .

8. System (200) according to Claim 7, wherein the second voltage supply (152) is supplied with electrical energy via a medium conducting communication signals which is arranged between the second communication interface belonging to the communication network (118) and the apparatus for converting electrical data signals into optical data signals and/or for converting optical data signals into electrical data signals.

## Revendications

1. Appareil de technique médicale (100) comprenant un boîtier (102), des composants électriques et/ou électroniques disposés à l'intérieur du boîtier (102), et une interface utilisateur (104),
l'appareil de technique médicale (100) possédant en outre un premier dispositif d'accueil (114) d'une première interface de communication (106-108), accessible depuis l'extérieur du boîtier (102) et destiné à accueillir une extrémité proximale d'un support (116) qui conduit des signaux de communication,
une extrémité distale du support (116) qui conduit des signaux de communication étant prévue pour le raccordement à un deuxième dispositif d'accueil (120), lequel est relié par le biais d'un réseau de communication (118) ne faisant pas partie de l'appareil de technique médicale (100) à une deuxième interface de communication ne faisant pas partie de l'appareil de technique médicale (100),
un trajet de communication entre le réseau de communication (118) et des composants électriques de l'appareil de technique médicale (100), se trouvant en liaison de communication avec la première interface de communication, possédant au moins deux segments,
et au moins l'un des segments transmettant des signaux de communication sans conduction électrique et présentant une séparation électrique entre l'entrée et la sortie ou entre la sortie et l'entrée du segment avec une première rigidité diélectrique prédéfinie,
l'au moins un segment du trajet de communication, lequel transmet des signaux de communication sans conduction électrique, étant disposé à l'intérieur du boîtier (102) de l'appareil de technique médicale (100) et un premier circuit (130) destiné à convertir des signaux électriques en optiques et/ou des signaux optiques en électriques se trouvant sur le ou dans l'appareil (100), **caractérisé en ce que** le premier circuit (130) est alimenté en énergie électrique par une source de tension (138 ; SR) disposée à l'extérieur de l'appareil de technique médicale, la tension fournie étant inférieure à une tension maximale prédéterminée considérée comme sûre.

2. Appareil de technique médicale (100) selon la revendication 1, l'au moins un segment du trajet de communication, lequel transmet des signaux de communication sans conduction électrique, transmettant les signaux de communication optiquement.

3. Appareil de technique médicale (100) selon la revendication 2, la transmission optique des signaux de communication s'effectuant dans un spectre qui comprend la lumière infrarouge et/ou visible.

4. Appareil de technique médicale (100) selon la revendication 1, un premier circuit (130) alimenté par une première source de tension (136) et destiné à convertir des signaux électriques en optiques et/ou des signaux optiques en électriques se trouvant sur le ou dans l'appareil de technique médicale (100), la première source de tension (136) garantissant une isolation électrique du premier circuit (130) destiné à convertir des signaux électriques en optiques et/ou des signaux optiques en électriques d'autres composants électriques et/ou électroniques internes à l'appareil avec une rigidité diélectrique qui correspond au moins à la première rigidité diélectrique prédéfinie.

5. Appareil de technique médicale (100) selon la revendication 1, un premier circuit (130) destiné à convertir des signaux électriques en optiques et/ou des signaux optiques en électriques se trouvant sur le ou dans l'appareil (100), lequel est alimenté en énergie électrique par le biais du support (116) qui conduit des signaux de communication, une tension d'alimentation fournie par ce biais étant inférieure à une tension maximale prédéterminée considérée comme sûre.

6. Appareil de technique médicale (100) selon la revendication 1 ou 2, le premier dispositif d'accueil étant conçu pour accueillir un support qui conduit des signaux de communication optiques.

7. Système (200) comprenant un appareil de technique médicale (100) selon la revendication 1,
le premier dispositif d'accueil (134) accessible depuis l'extérieur du boîtier (102) de l'appareil de technique médicale (100) étant conçu pour accueillir une extrémité proximale d'un support (132) qui conduit des signaux de communication optiques,
le système (200) comprenant en outre le support (132) qui conduit des signaux de communication optiques,
le système (200) comprenant, à l'extrémité distale du support (132) qui conduit des signaux de communication optiques, un dispositif (150) destiné à convertir des signaux électriques en optiques et/ou des signaux optiques en électriques avec une deuxième source de tension (152) et
la deuxième source de tension étant alimentée en énergie par une source d'énergie qui se trouve à l'extérieur de l'appareil de technique médicale (100).

8. Système (200) selon la revendication 7, la deuxième source de tension (152) étant alimentée en énergie électrique par le biais d'un support qui conduit des signaux de communication et qui est disposé entre la deuxième interface de communication faisant partie du réseau de communication (118) et le dispositif destiné à convertir des signaux de données électriques en signaux de données optiques et/ou des signaux de données optiques en signaux de données électriques.
